# DEMANDE DE BREVET EUROPEEN

(11) **EP 1 442 739 A1**
(43) Date de publication de la demande: **04.08.2004**
(21) Numéro de dépôt: 04290253.6
(22) Date de dépôt: 30.01.2004
(51) Int. Cl.: A61K 7/48, A61K 31/375, A61P 17/00

(54) **Utilisation de mono- ou di-esters d'acide cinnamique ou de l'un de ses dérivés et de vitamine C, comme donneur de NO.**

(30) Priorité: 03.02.2003 FR 0301210; 06.03.2003 US 452054 P
(71) Demandeur: L'OREAL, 75008 Paris (FR)
(72) Inventeur: Cals-Grierson, Marie-Madeleine, 92190 Meudon (FR)
(74) Mandataire: Vaillant, Jeanne

(57) **Abrégé**

La présente invention concerne l'utilisation de mono- ou di-esters d'acide cinnamique ou de l'un de ses dérivés et de vitamine C, en particulier le 2-O-cinnamate d'ascorbyle, pour augmenter la production de monoxyde d'azote dans ou sur la peau ou le cuir chevelu ou les muqueuses ainsi qu'un procédé d'application de monoxyde d'azote dans ou sur la peau, le cuir chevelu ou les muqueuses. Elle concerne également un procédé de traitement cosmétique mettant en oeuvre ces composés, en tant que donneurs de NO.

## Description

La présente invention concerne l'utilisation de mono- ou di-esters d'acide cinnamique ou de l'un de ses dérivés et de vitamine C pour augmenter la production de monoxyde d'azote dans ou sur la peau ou le cuir chevelu ou les muqueuses ainsi qu'un procédé d'application de monoxyde d'azote dans ou sur la peau, le cuir chevelu ou les muqueuses.

Dans le domaine de la santé et de la cosmétique, l'importance du monoxyde d'azote NO est connue notamment pour le rôle protecteur du NO produit par les synthases du monoxyde d'azote NOS ("Nitric Oxide Synthases" ), sous les trois isoformes NOS1 et NOS3 constitutives, et NOS2, inductible. NO est en effet impliqué dans de nombreux processus biologiques, notamment dans le système immunitaire, et il a de nombreuses interactions avec les acides nucléiques, les protéines, les thiols à bas poids moléculaire, etc.

Des applications topiques de monoxyde d'azote ont été décrites ainsi que des compositions complexes capables d'en générer. Ainsi, le brevet US 6 103 275 décrit des compositions comportant deux gels dont l'un comporte un sel, comme du nitrite de sodium, et l'autre comporte de l'acide ascorbique et de l'acide maléique, les deux gels étant mis en contact avant leur application, le monoxyde d'azote est généré dans la composition résultante, à même la peau.

Toutefois ces compositions présentent des inconvénients liés notamment au mode d'application et à leur formulation complexe à mettre en oeuvre.

On constate donc qu'il subsiste un besoin d'actif que l'on peut utiliser par voie orale ou topique, efficace pour la production de NO dans ou sur la peau, le cuir chevelu ou les muqueuses, en usage préventif ou curatif, facile à formuler, appliquer ou ingérer, et dépourvu d'effets secondaires.

Les composés de formule I ci-dessous sont décrits comme antioxydants et notamment protecteurs à l'égard de l'oxydation des constituants lipidiques de la peau et décrits dans des compositions pharmaceutiques, cosmétiques ou alimentaires, dans le document EP 664 290.

De façon surprenante, la demanderesse a maintenant mis en évidence que ces dérivés de mono- ou di-esters d'acide cinnamique ou de l'un de ses dérivés et de vitamine C sont particulièrement efficaces pour produire du monoxyde d'azote ou pour augmenter la production de monoxyde d'azote dans la peau, les muqueuses ou le cuir chevelu.

En effet, la Demanderesse a pu mettre en évidence un effet stimulateur de ces composés sur la production de NO, dans le test de screening de modulation de l'induction de NOS2 par les kératinocytes humains normaux.

Les produits de l'art antérieur connus comme donneurs de NO agissent en libérant in situ le NO, après une réaction chimique ou clivage enzymatique. L'activité de ces composés impliquerait un mécanisme différent, en augmentant une voie de production endogène.

L'invention a pour objet l'utilisation de dérivés mono- ou di-esters d'acide cinnamique ou de l'un de ses dérivés et de vitamine C pour augmenter la production de monoxyde d'azote dans ou sur la peau, les muqueuses ou le cuir chevelu.

Les composés utilisés conformément à l'invention pour augmenter la production de monoxyde d'azote dans la peau ou en obtenir une production efficace sont les composés de formule I 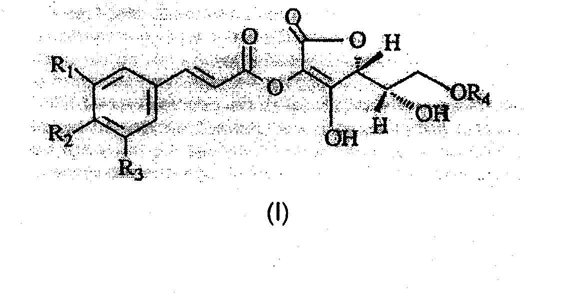 dans laquelle :
R₁,R₂ et R₃, indépendamment l'un de l'autre, représentent H ou un radical OH, alkoxy, fluoroalkoxy ou alkylcarbonyloxy, et
R₄ représente H ou -COR₅ , R₅ représentant un radical alkyle linéaire ou ramifié, en C₁ à C₂₀ ou le radical de formule : 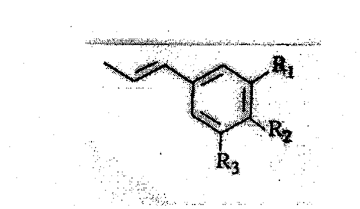 ainsi que les substances apparentées ou pouvant en générer, leurs analogues et précurseurs.

Le radical alkoxy est de préférence un radical méthoxy, éthoxy ou butoxy ; le radical fluoroalkoxy est de préférence le radical trifluorométhoxy et le radical alkylcarbonyloxy est de préférence un radical acétoxy, propionyloxy ou butyryloxy.

De façon préférée, on utilise le 2-O-cinnamate d'ascorbyle, le 2-O-férulate d'ascorbyle, le 2-O-caféate d'ascorbyle, le 2-O-sinapate d'ascorbyle, le 4'-acétoxy-férulate de 2-O-[6-palmitoyl-ascorbyle], et de façon encore préférée le 2-O-cinnamate d'ascorbyle.

Selon l'invention, ces composés sont utilisés comme actifs, cosmétiques et/ou pharmaceutiques, pour augmenter la production de monoxyde d'azote dans ou sur la peau, les muqueuses ou le cuir chevelu, l'application étant réalisée par voie topique ou orale, notamment, et l'invention concerne l'utilisation des composés ci-dessus pour la préparation de compositions comportant au moins l'un des composés, sels ou complexes métalliques acceptables et un excipient acceptable, destinée au traitement des matières kératiniques et des muqueuses, par production de NO ou par augmentation de la production de NO.

Ainsi, ces composés peuvent être utilisés plus particulièrement pour la préparation des compositions destinées à favoriser la vascularisation de la peau ou des muqueuses et/ou la revascularisation des zones lésionnelles psoriatiques et/ou des ulcères chroniques, et/ou pour limiter le prurit et/ou l'eczéma. Ils peuvent en outre être utilisés pour réguler la croissance des bactéries cutanées, notamment contre le développement d'odeur liées à la sueur.

En outre, ils peuvent être utilisés dans des compositions utiles pour protéger des dommages provoqués par des ultraviolets induits, comme des érythèmes, et/ou pour favoriser la mélanogénèse et la pigmentation cutanée et/ou capillaire. De plus, ils peuvent être utilisés pour moduler la croissance du poil ou du cheveu ; par exemple dans des compositions antichute.

Ils peuvent en outre être utilisés dans des préparations cosmétiques de soin anti-âge destinées à limiter le dépôt de collagène hautement réticulé, et/ou destinées à favoriser la synthèse du collagène. De plus, ils peuvent être utilisés dans des compositions destinées à favoriser la lipolyse, et ainsi faciliter l'amincissement. De plus, ils peuvent être utilisés dans des compositions cosmétiques destinées à moduler la pigmentation des cheveux. Ils peuvent aussi être utilisés dans des compositions cosmétiques utiles à la régularisation de la sudation et des odeurs correspondantes. Ils peuvent aussi être utilisés dans des compositions cosmétiques utiles à la régulation des peaux grasses. Enfin, ils peuvent être utilisés dans des compositions cosmétiques en tant que myorelaxant.

Dans des applications pharmaceutiques, les composés de l'invention sont utiles pour préparer les compositions destinées à la lutte contre les mycoses cutanées et/ou la lutte contre le lupus érythémateux. Les composés peuvent encore être utilisés pour la préparation de compositions pharmaceutiques utiles pour favoriser la microcirculation et lutter notamment contre le syndrome de Raynaud et/ou prévenir ou réduire la prolifération épidermique notamment durant la cicatrisation. Enfin, ils peuvent être utilisés pour la préparation de composition pharmaceutiques utiles pour réguler le système immunitaire.

Les compositions cosmétiques et pharmaceutiques comportent notamment au moins un composé de formule I ou l'un des sels ou complexes métalliques acceptables, dans un milieu cosmétiquement ou pharmaceutiquement acceptable.

Selon l'invention, le ou les composés sont présents dans ces compositions dans des proportions allant de 0,001 à 10 % en poids.

Les compositions cosmétiques et pharmaceutiques peuvent se présenter sous des formes diverses habituellement utilisées dans ces domaines pharmaceutiques et cosmétiques ou de l'hygiène corporelle, et en particulier sous forme d'onguent, de crème, de pommade, de comprimé, de suspension buvable, d'injection ou de gel, par exemple, pour les compositions pharmaceutiques et sous forme de gel, de spray, de lotion, d'émulsion ou de dispersion vésiculaire, par exemple, pour les compositions cosmétiques, ainsi que sous forme de compléments alimentaires, sans que ces formes particulières soient limitatives pour les applications cosmétiques ou pharmaceutiques respectivement.

Lorsque les composés de formule (I) sont utilisés dans le cadre d'un traitement pharmaceutique, les formes d'administration peuvent adopter la voie orale, topique, entérale ou parentérale, le support pharmaceutiquement acceptable dépendant de la forme d'administration choisie. La posologie est généralement comprise entre 1 et 100 mg/kg/jour.

Le milieu cosmétiquement ou pharmaceutiquement acceptable est un milieu usuel dans le domaine cosmétique ou pharmaceutique.

Lorsque les composés de formule I sont utilisés dans le cadre d'un traitement cosmétique, les formes d'application peuvent adopter non seulement la voie topique, mais encore la voie orale.

L'invention sera mieux comprise à la lecture de la description détaillée et des exemples suivants.

Les composés de formule I peuvent être préparés selon les procédés décrits dans le document EP 664 290.

Selon l'invention, on préfère la voie topique, par application directe sur les matières kératiniques, comme la peau, le cuir chevelu, les ongles ou sur les muqueuses.

Les compositions selon l'invention peuvent toutefois se présenter sous toutes les formes galéniques et cosmétiques compatible avec leur rôle de producteur de NO, notamment les formes décrites dans EP 664 290, ou toute autre forme, y compris celle de compléments alimentaires.

Ces compositions sont préparées selon les méthodes usuelles.

Un milieu cosmétiquement ou dermatologiquement acceptable correspond généralement à un milieu compatible avec les matières kératiniques, la peau, le cuir chevelu, les ongles ou les muqueuses. La composition comprenant le ou les actifs donneurs de NO peut donc être appliquée sur le visage, le cou, les cheveux et les ongles, ou toute autre zone cutanée du corps (régions axillaires, sous-mammaires, plis du coude etc.).

Par voie topique, les compositions selon l'invention se présentent notamment sous forme de solutions aqueuses, hydroalcooliques ou huileuses, de dispersions du type lotion ou sérum, de gels anhydres ou lipophiles, d'émulsions de consistance liquide ou semi-liquide du type lait, obtenues par dispersion d'une phase grasse dans une phase aqueuse (H/E) ou inversement (E/H), ou de suspensions ou d'émulsions de consistance molle, semi-solide ou solide du type crème ou gel, ou encore de microémulsions, de microcapsules, de microparticules ou de dispersions vésiculaires de type ionique et/ou non ionique. Ces compositions sont préparées selon les méthodes usuelles.

Par voie entérale, les compositions selon l'invention peuvent se présenter sous forme de comprimés, de gélules, de dragées, de sirops, de suspensions, de solutions, de microsphères ou de nanosphères ou de vésicules lipidiques ou polymériques permettant une libération contrôlée.

Par voie parentérale, les compositions peuvent se présenter sous forme de solutions ou de suspensions pour perfusion ou pour injection.

Elles peuvent être également utilisées pour le cuir chevelu sous forme de solutions aqueuses, alcooliques ou hydroalcooliques, ou sous forme de crèmes, de gels, d'émulsions, de mousses ou encore sous forme de compositions pour aérosol contenant également un agent propulseur sous pression.

Les quantités des différents constituants des compositions selon l'invention sont celle classiquement utilisées dans les domaines considérés.

Ces compositions constituent notamment des mousses à raser, des crèmes de nettoyage, de protection, de traitement ou de soin pour le visage, pour les mains, pour les pieds, pour les grands plis anatomiques ou pour le corps, (par exemple crèmes de jour, crèmes de nuit, crèmes démaquillantes, crèmes de fond de teint, crèmes anti-solaires), des fond de teint fluides, des laits de démaquillage, des laits corporels de protection ou de soin, des laits anti-solaires ou mieux après solaires, des lotions, gels ou mousses pour le soin de la peau, comme des lotions de nettoyage ou de désinfection, des lotions anti-solaires, des lotions de bronzage artificiel, des compositions pour le bain, des compositions désodorisantes contenant un agent bactéricide, des gels ou lotions après-rasage, des crèmes épilatoires, des compositions contre les piqûres d'insectes, des compositions anti-douleur ou des compositions pour traiter certaines maladies notamment de la peau comme celle citées précédemment.

Les compositions selon l'invention peuvent également consister en des préparations solides constituant des savons ou des pains de nettoyage.

Les compositions peuvent aussi être conditionnées sous forme de composition pour aérosol contenant également un agent propulseur sous pression.

Les composés utilisés selon l'invention peuvent être aussi incorporés dans diverses compositions pour soins ou traitement capillaires, et notamment des shampooings éventuellement antiparasitaires, des lotions de mise en plis, des lotions traitantes, des crèmes ou des gels coiffants, des compositions de teinture (notamment teintures d'oxydation) éventuellement sous forme de shampooings colorants, des lotions restructurantes pour les cheveux, des compositions pour le premier temps d'une permanente), des lotions ou des gels antichute, etc.

Les compositions de l'invention peuvent aussi être à usage bucco-dentaire, par exemple une pâte dentifrice ou un bain de bouche. Dans ce cas, les compositions peuvent contenir des adjuvants et additifs usuels pour les compositions à usage buccal et notamment des agents tensioactifs, des agents épaississants, des agents humectants, des agents de polissage tels que la silice, divers ingrédients actifs comme les fluorures, en particulier le fluorure de sodium, et éventuellement des agents édulcorants comme le saccharinate de sodium.

Lorsque la composition de l'invention est une émulsion, la proportion de la phase grasse peut aller de 5 % à 80 % en poids, et de préférence de 5 % à 50 % en poids par rapport au poids total de la composition. Les huiles, les émulsionnants et les coémulsionnants utilisés dans la composition sous forme d'émulsion sont choisis parmi ceux classiquement utilisés dans les domaines cosmétiques et pharmaceutique. L'émulsionnant et le coémulsionnant sont présents, dans la composition, en une proportion allant de 0,3 % à 30 % en poids, et de préférence de 0,5 à 30 % ou mieux de 0,5 à 20 % en poids par rapport au poids total de la composition. L'émulsion peut, en outre, contenir des vésicules lipidiques.

Lorsque la composition de l'invention est une solution ou un gel huileux, la phase grasse peut représenter plus de 90 % du poids total de la composition.

De façon connue, la composition de l'invention peut contenir également des adjuvants habituels dans le domaine cosmétique ou pharmaceutique, tels que les gélifiants hydrophiles ou lipophiles, les actifs hydrophiles ou lipophiles, les conservateurs, les antioxydants, les solvants, les parfums, les charges, les filtres, les bactéricides, les absorbeurs d'odeur et les matières colorantes. Les quantités de ces différents adjuvants sont celles classiquement utilisées dans le domaine cosmétique ou pharmaceutique, et par exemple de 0,01 % à 10 % du poids total de la composition. Ces adjuvants, selon leur nature, peuvent être introduits dans la phase grasse, dans la phase aqueuse et/ou dans les sphérules lipidiques.

Comme huiles utilisables dans l'invention, on peut citer les huiles minérales (huile de vaseline), les huiles végétales (fraction liquide du beurre de karité, huile de tournesol), les huiles animales (perhydrosqualène), les huiles de synthèse (huile de Purcellin), les huiles siliconées (cyclornéthicone) et les huiles fluorées (perfluoropolyéthers). On peut aussi utiliser comme matières grasses des alcools gras, des acides gras (acide stéarique), des cires (paraffine, carnauba, cire d'abeilles).

Comme émulsionnants utilisables dans l'invention, on peut citer par exemple le stéarate de glycérol, le polysorbate 60 et le mélange de PEG-6/PEG-32/Glycol Stéarate vendu sous la dénomination de Tefose^{R} 63 par la société Gattefosse.

Comme solvants utilisables dans l'invention, on peut citer les alcools inférieurs, notamment l'éthanol et l'isopropanol, le propylène glycol.

Comme gélifiants hydrophiles, on peut citer les polymères carboxyvinyliques (carbomer), les copolymères acryliques tels que les copolymères d'acrylates/alkylacrylates, les polyacrylamides, les polysaccharides tels que l'hydroxypropylcellulose, les gommes naturelles et les argiles, et, comme gélifiants lipophiles, on peut citer les argiles modifiées comme les bentones, les sels métalliques d'acides gras comme les stéarates d'aluminium et la silice hydrophobe, ou encore l'éthylcellulose, le polyéthylène.

Les composés utilisés selon l'invention peuvent être utilisés en association avec d'autres actifs.

Comme actif hydrophiles, on peut utiliser les protéines ou les hydrolysats de protéine, les acides aminés, les polyols, l'urée, l'allantoïne, les sucres et les dérivés de sucre, les vitamines hydrosolubles, l'amidon et des extraits végétaux, notamment ceux d'Aloe Vera, par exemple.

Comme actifs lipophiles, on peut utiliser le rétinol (vitamine A) et ses dérivés, le tocophérol (vitamine E) et ses dérivés, les acides gras essentiels, les céramides, les huiles essentielles, ou les polyphénols, par exemple.

On peut en outre associer les composés donneurs de NO selon l'invention à d'autres agents actifs donneurs de NO ou actifs destinés notamment à la prévention et/ou au traitement des affections cutanées, mucosales et/ou capillaires.

Parmi ces agents actifs, on peut citer à titre d' exemple les N-arylméthylèneéthylènediaminetriacétates, N-arylméthylène-iminodiacétates ou N,N'-diarylméthylène éthylènediamineacétates, et leurs sels et complexes métalliques et de façon préférée l'acide NN'-bis-(3, 4, 5-triméthoxybenzyl) éthylènediamine-diacétique, ses analogues et précurseurs. Comme sels, on peut citer les sels d'addition avec un acide minéral comme les acides H₂SO₄, HCl, HNO₃ ou H₃PO₄, par exemple, et les sels d'addition avec une base minérale comme NaOH ou KOH. Comme complexes métalliques, on peut citer les complexes formés par addition de ZnCl₂ ou CaCl₂, par exemple.

Par ailleurs, d'autres donneurs de NO peuvent être associés. Parmi ceux-ci, par exemple les dérivés nitro et S-nitroso d'anti-inflammatoires non-stéroïdiens, (NO-NSAIDs) et de stéroïdiens (NO-stéroïdes), usuellement obtenus par ajout d'un groupe ONO₂ par une liaison ester à la molécule anti-inflammatoire parente. Ces composés sont connus sous les appellations de NO-aspirine, NO-paracétamol, NO-fulbiprofen, par exemple.

Plus généralement, peuvent être associés aux composés utilisés selon l'invention des donneurs de NO directs ou des donneurs de NO nécessitant un métabolisme pour la production de NO, que leur mécanisme de production agisse sur l'activité de donneur endogène ou exogène.

La présente invention a en outre pour objet un procédé de traitement cosmétique, qui met en oeuvre le ou les composé(s) ou la composition cosmétique ci-dessus, notamment pour les utilisations décrites ci-dessus. On peut appliquer sur la peau, les muqueuses et/ou le cuir chevelu les compositions cosmétiques, ou encore ingérer des compositions prévues à cette fin.

Préférentiellement, le procédé de traitement cosmétique consiste à appliquer sur la peau, sur le cuir chevelu, et/ou sur les muqueuses, une composition telle que décrite ci-dessus. Ainsi, le procédé de traitement cosmétique de l'invention peut être mis en oeuvre notamment en appliquant les compositions hygiéniques ou cosmétiques telles que définies ci-dessus, selon la technique d'utilisation habituelle de ces compositions, et par exemple : application de crèmes, de gels, de sérums, de lotions, de laits de démaquillage ou de compositions après-solaires sur la peau ou sur les cheveux mouillés, de shampooings ou encore application de dentifrice sur les gencives.

### Exemple 1 : Activité biologique du 2-O-cinnamate d'ascorbyle

L'activité du 2-O-cinnamate d'ascorbyle sur la NO-synthase inductible a été évalué dans le test décrit par Heck et col. (J.B.C., Vol. 267, N° 30, 21277-21280, 25 octobre 1992), test de screening de modulation d'induction de NOS2 par les keratinocytes humains normaux. Ce test a pour objectif d'évaluer la concentration en nitrate et nitrite, in fine, après stimulation de la NO-synthase 2.

Le test est effectué sur une culture de kératinocytes humains normaux, issus de prélèvements. L'induction de la NO synthase inductible (NOS2) a été provoquée par l'addition d'une combinaison de plusieurs cytokines au milieu de culture. Le produit testé a été appliqué à trois concentrations variant de 10 à 1000 µM.

Le produit testé a été classé dans une des deux catégories suivantes : inhibiteurs (I) ou donneurs (D) de NO (monoxyde d'azote) selon sa faculté à diminuer ou à augmenter, respectivement, la quantité de nitrites et nitrates produits par rapport au signal témoin (sans cytokines).

Les contrôles suivants ont été introduits dans le test :
A : contrôle positif (induction de l'enzyme) : mélanges d'interféron-γ (1000 u/ml) et d'interleukine 1-β (100 u/ml)
B : contrôle négatif (inhibition maximale) : N⁹-monométhyl-L-argine (forme L) à 200 µm ;
C : contrôle de spécificité de l'inhibition : N⁹-monométhyl-L-arginine (forme D) à 200 µm.

Pour déterminer l'activité du produit à tester on mesure la quantité de produits de réaction stables du NO (nitrites et nitrates) à l'aide du kit "nitric colorimetric assay" vendu par la société Boehringer sous la référence 1756.28.

Le 2-O-cinnamate d'ascorbyle a été testé aux concentrations de 100 µM, 500 µM et 1 000 µM dans l'éthanol.

| Produit testé | % inhibition |
|---|---|
| A | 0 |
| B | 100 |
| C | 0 |
| 2-O-cinnamate d'ascorbyle : 100 µM | 24,79 % |
| 2-O-cinnamate d'ascorbyle : 500 µM | 53,74 % |
| 2-O-cinnamate d'ascorbyle : 1000 µM | 74,4% |

On conclut que le 2-O-cinnamate d'ascorbyle présente un effet donneur de NO.

Par ailleurs, la cytotoxicité a été exprimée en % de diminution du signal par rapport au témoin. Un produit a été considéré comme peu cytotoxique de 0-20 %, acceptable jusqu'à 40 % et non retenu pour des valeurs > 40 %.

On a également conclu que le produit n'a pas de toxicité.

## Revendications

1. Utilisation d'au moins un composé de formule I 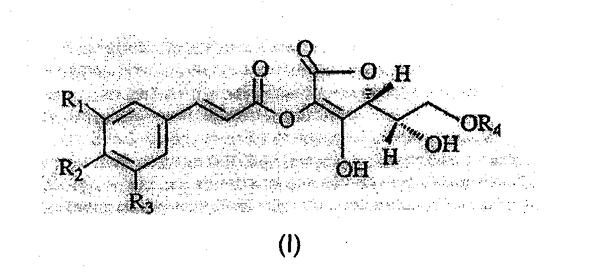 dans laquelle :
R₁,R₂ et R₃, indépendamment l'un de l'autre, représentent H ou un radical OH, alkoxy, fluoroalkoxy ou alkylcarbonyloxy, et
R₄ représente H ou -COR₅ , R₅ représentant un radical alkyle linéaire ou ramifié, en C₁ à C₂₀ ou le radical de formule : 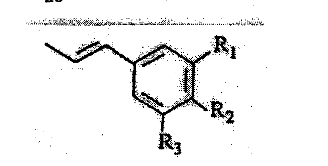 pour la préparation d'une composition comportant au moins un composé de formule I et un excipient acceptable, destinée au traitement des matières kératiniques et des muqueuses par production de monoxyde d'azote ou augmentation de la production de monoxyde d'azote dans ou sur la peau, les muqueuses ou le cuir chevelu.

2. Utilisation selon la revendication 1, **caractérisée en ce que** la composition est utile pour favoriser la vascularisation de la peau ou des muqueuses.

3. Utilisation selon l'une des revendications 1 à 2, **caractérisée en ce que** la composition est utile pour revasculariser des zones lésionnelles psoriatiques et/ou des ulcères chroniques.

4. Utilisation selon l'une des revendications 1 à 3, **caractérisée en ce que** la composition est utile pour limiter le prurit et/ou l'eczéma.

5. Utilisation selon l'une des revendications 1 à 4, **caractérisée en ce que** la composition est utile pour protéger des dommages provoqués par des ultraviolets, comme des érythèmes.

6. Utilisation selon l'une des revendications 1 à 5, **caractérisée en ce que** la compositicn est utile pour moduler la croissance du poil ou du cheveu.

7. Utilisation selon l'une des revendications 1 à 6, **caractérisée en ce que** la composition est utile pour favoriser la mélanogénèse et la pigmentation cutanée et/ou capillaire.

8. Utilisation selon l'une des revendications 1 à 7, **caractérisée en ce que** la composition est utilisée pour réguler la croissance des bactéries cutanées.

9. Utilisation selon la revendication 1 à 8, **caractérisée en ce que** la composition est cosmétique et destinée à limiter la chute des cheveux.

10. Utilisation selon l'une des revendications 1 à 9, **caractérisée en ce que** la composition cosmétique est utile pour moduler la pigmentation des cheveux.

11. Utilisation selon l'une des revendications 1 à 10, **caractérisée en ce que** la composition cosmétique est une composition de soin anti-âge destinée à favoriser la synthèse du collagène et/ou destinées à limiter le dépôt de collagène hautement réticulé.

12. Utilisation selon l'une des revendications 1 à 11, **caractérisée en ce que** la composition cosmétique est utile dans des préparations pour favoriser la lipolyse.

13. Utilisation selon l'une des revendications 1 à 12, **caractérisée en ce que** la composition cosmétique est utile pour favoriser la régulation des peaux grasses.

14. Utilisation selon l'une des revendications 1 à 13, **caractérisée en ce que** la composition cosmétique est utile pour favoriser la régulation de la sudation et odeurs correspondantes.

15. Utilisation selon l'une des revendications 1 à 14, **caractérisée en ce que** la composition est utilisée dans des applications cosmétiques en tant que myorelaxant.

16. Utilisation selon l'une des revendications 1 à 8, **caractérisée en ce que** la composition pharmaceutique est utile dans un traitement de lutte contre les mycoses cutanées.

17. Utilisation selon l'une des revendications 1 à 8 et 16, **caractérisée en ce que** la composition pharmaceutique est utile dans un traitement de lutte contre le lupus érythémateux.

18. Utilisation selon l'une des revendications 1 à 8 et 16 ou 17, **caractérisée en ce que** la composition pharmaceutique est utile dans un traitement pour favoriser la microcirculation et/ou lutter contre le syndrome de Raynaud.

19. Utilisation selon l'une des revendications 1 à 8 et 16 à 18, **caractérisée en ce que** la composition pharmaceutique est utile dans un traitement de prévention ou de réduction de la prolifération épidermique.

20. Utilisation selon l'une des revendications 1 à 8 et 16 à 19, **caractérisée en ce que** la composition pharmaceutique est utile dans un traitement de régulation du système immunitaire.

21. Utilisation selon l'une des revendications 1 à 8 et 16 à 20, **caractérisée en ce que** la composition pharmaceutique est utile pour limiter la chute des cheveux et favoriser leur repousse.

22. Utilisation selon l'une des revendications 1 à 21, **caractérisée en ce que** le produit de formule 1 est le 2-O-cinnamate d'ascorbyle .

23. Utilisation pour le traitement cosmétique de l'organisme, en vue de la production ou l'augmentation de la production de monoxyde d'azote dans ou sur la peau, les cheveux ou les muqueuses, d'un composé de formule I définie à la revendication 1 ou 22.

24. Utilisation comme donneur de NO d'un composé de formule I définie à la revendication 1 ou 22.

25. Procédé de traitement cosmétique consistant à appliquer sur la peau, les muqueuses et/ou le cuir chevelu ou à ingérer une composition comportant au moins ur composé de formule 1 définie à la revendication 1.

26. Procédé selon la revendication 25, dans lequel la composition comporte au moins du 2-O-cinnamate d'ascorbyle.
